# NEW EUROPEAN PATENT SPECIFICATION

(11) **EP 0 818 192 B2**
(45) Date of publication and mention of the opposition decision: **23.08.2006**
(45) Mention of the grant of the patent: 04.11.1998
(21) Application number: 97111884.9
(22) Date of filing: 12.07.1997
(51) Int. Cl.: A61K 8/34, A61K 8/41, A61K 8/49, A61Q 5/10

(54) **Hair dyeing composition**
Haarfärbemittel
Composition pour la teinture des cheveux

(30) Priority: 13.07.1996 DE 19628344; 13.07.1996 DE 19628343
(43) Date of publication of application: 14.01.1998
(73) Proprietor: KPSS-Kao Professional Salon Services GmbH, 64297 Darmstadt (DE)
(72) Inventor: Lorenz, Heribert, 64401 Gross-Bieberau (DE); Golinski, Frank, Dr., 64297 Darmstadt (DE)

(56) References cited:
- EP-A- 0 370 880
- EP-A- 0 657 159
- EP-A- 0 766 958
- EP-A- 0 782 845
- US-A- 5 494 489

## Description

This invention refers to a hair dyeing composition on the basis of a developing/coupling agent system reacting with peroxides, providing an expressive, brilliant and durable orange/copper brown-red basic coloring which may be used either as such, or in combination with other developing and (or) coupling agents to achieve additional color shades.

The developing agents most frequently used in hair dyeing compositions are still 1,4-diaminobenzene (p-phenylenediamine) and 1-methyl-2,5-diaminobenzene (p-toluylenediamine). The application of these substances in combination with various coupling agents allows the creation of numerous color shades.

A high variety of color shades is also provided by the use of triaminohydroxypyrimidines known from European Patent No. 467 026, particularly 2,5,6-triamino-4-hydroxypyrimidine, 2,4,5-triamino-6-hydroxypyrimidine, 4,5,6-triamino-2-hydroxypyrimidine or their salts, especially sulfate salts, as developing agents in hair dyeing compositions.

Document EP-A-0 657 159 discloses a hair dyeing composition.

However, there is still a need for improvement, particularly in the range of orange/copper red colors. The invention, therefore, starts from the problem to provide oxidation hair dyeing compositions which lead to an expressive orange/copper red color which may be used either as such or in combination with further coupling agents or shade modifiers to produce additional color shades.

According to the invention, this problem is solved by using a combination of developing and coupling agents reacting with peroxides, comprising a triaminohydroxypyrimidine, or the water-soluble salts thereof, and a mixture of 4-amino-3-methyl phenol and 2-amino-3-hydroxypyridine.

The use of such a product gives stable orange or copper red-brown color shades.

The invention is defined by the claims.

The weight proportion of 4-amino-3-methyl phenol to 2-amino-3-hydroxypyridine is preferably 1 : 10 to 10 : 1, particularly 1 : 5 to 5 : 1, optimally 1 : 2 to 2 : 1 e.g. 1 : 1.

The hair dyeing compositions of the invention may contain further coupling agents. These are, e.g., preferably resorcinol, 2-methyl resorcinol, 2-chlororesorcinol, m-aminophenol, m-phenylenediamine, α-naphthol, p-amino-4-(hydroxyethyl)-aminoanisole, o-chloro-p-phenylenediamine, 1,7-dihydroxynapnthaline and 3-dimethyl aminophenol.

In addition to the developing agents according to the invention, further known developing agents may be used such as 1,2,4-triaminobenzene, tetraaminopyrimidines, 1-(β-hydroxyethyl)-2,5-diaminobenzene (cf. European Patent Application No.7537 and European Patent No. 400 330), 1-amino-4-bis-(2'-hydroxyethyl)aminobenzene, and (or) 2-(2'-hydroxyethyl amino)-5-aminotoluene (cf. European Patent Application No. 615 743). These compounds as well as 1,4-diaminobenzene and 1-methyl-2,5-diaminobenzene are preferably used as water-soluble salts, but they may also, of course, be used as free bases.

The proportion of developing agents in the hair dyeing compositions according to the invention is between about 0.05% and 5%, preferably 0.1% and 4%, particularly 0.5% and 3% by wt. of the total composition (excluding oxidizing agents).

The coupling agents in the hair dyeing compositions according to the invention are used in about the same ratio as the developing agents, i.e., in quantities from about 0.05% to 5%, preferably 0.1% to 4%, particularly 0.5% to 3% by wt., of the total composition (excluding oxidizing agents).

It is possible and often advisable to use additional known coupling agents if desired or necessary to provide specific color shades.

Optionally the compositions according to the invention may also contain so-called modifiers for the fine-adjustment of a desired shade, particularly direct-acting dyestuffs. These shade modifiers are e.g. nitro-dyestuffs such as 2-amino-4,6-dinitrophenol, 2-amino-4-nitrophenol, 2-amino-6-chloro-4-nitrophenol, etc., preferably in proportions from about 0.05% to 2.5%, particularly 0.1% to 1% by wt. of the dyeing composition (excluding oxidizing agents).

The hair dyeing compositions according to the invention may contain the usual carrier materials and additives, conditioning agents, etc., as they are known to the expert from the state of the art, and, e.g., described in the monography of K.Schrader, "Grundlagen und Rezepturen der Kosmetika", 2nd Ed. (1989, Hüthig Buchverlag, Heidelberg), pp. 782 to 815. They may be applied as solutions, creams, gels or aerosol compositions.

The oxidation hair dye precursor is mixed with an oxidizing agent before application onto the hair. A preferred oxidizing agent is hydrogen peroxide, e.g. in a 2% to 6% concentration.

However, other peroxides may also be used such as urea peroxide or melamin peroxide.

The pH-value of the ready-to-use hair dyeing composition, i.e. after admixture with peroxide, may be weakly acidic, i.e. from 5.5 to 6.9, or neutral, but also in the alkaline range, i.e. between 7.1 and 9.5.

A particularly gentle hair coloring treatment is achieved by the application of a weakly acidic hair dyeing composition.

The following examples illustrate some applications of the invention.

**Basic Formula**

| | |
|---|---|
| Stearyl alcohol | 8.0 (% by wt.) |
| Coconut fatty acid monoethanolamide | 4.5 |
| 1,2-Propanediol mono/distearate | 1.3 |
| Coconut fatty alcohol polyglycol ether | 4.0 |
| Sodium lauryl sulfate | 1.0 |
| Oleic acid | 2.0 |
| 1,2-Propanediol | 1.5 |
| Sodium EDTA | 0.5 |
| Sodium sulfite | 1.0 |
| Protein hydrolyzate | 0.5 |
| Ascorbic acid | 0.2 |
| Perfume | 0.4 |
| Ammonia, 25% | 8.5 |
| Ammonium chloride | 0.5 |
| Panfhenol | 0.8 |
| Water | @ 100.0 |

The combination of developing and coupling agents according to the invention was incorporated into this basic formula while reducing the water content accordingly.

The colorations were performed on wool patches and strands of bleached human hair by the application of a 1:1 mixture of hair dye precursor and 6% hydrogen peroxide solution, 20 minutes processing at room temperature, washing and drying.

### Example 1

| | |
|---|---|
| 0.27 (% by wt.) | 4-Hydroxy-2.5,6-triaminopyrimidine sulfate (TRAP) |
| 0.14 | 4-Amino-3-methyl phenol |
| 0.25 | 2-Amino-3-hydroxypyridine |
| Coloration: | Deep light-orange. |

### Comparisons:

### 1a):

| | |
|---|---|
| 0.27 (% by wt.) | TRAP |
| 0.14 | 4-Amino-3-methyl phenol |
| Coloration: | Weak light-yellow, not usable. |

### 1b):

| | |
|---|---|
| 0.27 (% by wt.) | TRAP |
| 0.25 | 2-Amino-3-hydroxypyridine |
| Coloration: | Weak light-yellow, not usable. |

## Claims

1. Hair dyeing composition, comprising a triaminohydroxypyrimidine, or the water-soluble salts thereof and one or more coupling agents, **characterized in that** it contains a combination of
a) 4-amino-3-methyl phenol and
b) 2-amino-3-hydroxypyridine.

2. Hair dyeing composition according to claim 1, comprising the mixture of 4-amino-3-methyl phenol with 2-amino-3-hydroxypyridine in a weight ratio from 10 : 1 to 1 : 10.

3. Hair dyeing composition according to claim 1 or 2, comprising at least one additional coupling agent selected from the group of resorcinol, 2-methyl resorcinol, 4-chlororesorcinol,1-methyl-2-hydroxy-4-aminobenzene, 3-aminophenol, α-naphthol, 3-amino-2-methyl amino-6-methoxypyridine and (or) 2-amino-4-(β-hydroxyethyl amino)anisole or the water-soluble salts thereof.

## Patentansprüche

1. Haarfärbemittel, enthaltend Triaminohydroxypyrimidin bzw. dessen wasserlösliche Salze und eine oder mehrere Kupplersubstanz(en), **dadurch gekennzeichnet, dass** es eine Kombination aus
a) 4-Amino-3-methylphenol und
b) 2-Amino-3-hydroxypyridin
enthält.

2. Haarfärbemittel nach Anspruch 1, **dadurch gekennzeichnet, dass** es das Gemisch aus 4-Amino-3-methylphenol und 2-Amino-3-hydroxypyridin in einem Gewichtsverhältnis von 10 : 1 bis 1 : 10 enthält.

3. Haarfärbemittel nach Anspruch 1 oder 2, **dadurch gekennzeichnet, dass** es mindestens eine zusätzliche Kupplersubstanz, ausgewählt aus der Gruppe Resorcin, 2-Methylresorcin, 4-Chlorresorcin, 1-Methyl-2-hydroxy-4-aminobenzol, 3-Aminophenol, α-Naphthol, 3-Amino-2-methylamino-6-methoxypyridin und/oder 2-Amino-4-(β-hydroxyethylamino)anisol bzw. dessen wasserlöslichen Salzen, enthält.

## Revendications

1. Composition pour la teinture des cheveux, comprenant une triaminohydroxypyrimidine ou ses sel hydrosolubles, et un ou plusieurs agent(s) de couplage, **caractérisée en ce qu'**elle contient une combinaison formée de
a) 4-amino-3-méthylphénol et
b) 2-amino-3-hydroxypyridine.

2. Composition pour la teinture des cheveux selon la revendication 1, contenant le mélange composé de 4-amino-3-méthylphénol et de 2-amino-3-hydroxypyridine selon un rapport pondéral compris entre 10 : 1 et 1 : 10.

3. Composition pour la teinture des cheveux selon la revendication 1 ou 2, contenant au moins un agent de couplage supplémentaire sélectionné parmi la groupe composé de résorcine, de 2-méthylrésorcine, de 4-chlororésorcine, de 1-méthyl-2-hydroxy-4-aminobenzène, de 3-aminophénol, d'α-naphtol, de 3-amino-2-méthylamino-6-méthoxypyridine et/ou de 2-amino-4-(β-hydroxyéthylamino)anisole ou ses sels hydrosolubles.
